(19) **Europäisches Patentamt**

**European Patent Office**

**Office européen des brevets**

(11) Publication number: **0 375 859**

**A2**

(12) # EUROPEAN PATENT APPLICATION

(21) Application number: **89119064.7**

(51) Int. Cl.5: **G01N 33/569**

(22) Date of filing: **13.10.89**

The title of the invention has been amended (Guidelines for Examination in the EPO, A-III, 7.3).

(30) Priority: **14.10.88 JP 259181/88**

(43) Date of publication of application:
**04.07.90 Bulletin 90/27**

(84) Designated Contracting States:
**AT BE CH DE FR GB IT LI NL SE**

(71) Applicant: **Eisai Co., Ltd.**
**6-10, Koishikawa 4-chome Bunkyo-ku Tokyo(JP)**

(72) Inventor: **Sawada, Takashi**
**9-17, Sengen 2-chome**
**Tsukuba-shi Ibaraki(JP)**
Inventor: **Tohmatsu, Junichi**
**10-26, Nakatakatsu 2-chome**
**Tsuchiura-shi Ibaraki(JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) **Method for detecting HTLV-I infection through mother's milk and reagents therefor.**

(57) HTLV-I infection is detected from a mother to a child through the mother's milk, comprising detecting an immunological roaction of the mother's serum to P40$^x$ using P40$^x$.

EP 0 375 859 A2

# Method for detecting infection through mother's milk and reagents therefor

The present invention relates to a method for detecting the presence or the occurrence of HTLV - I infection through mother's milk.

The virus responsible for adult T-cell leukamia (ATL) has been isolated as a C - type retrovirus from patients with adult T - cell leukemia and this virus has been identified as HTLV - I ( Human T lymphotrophic virus ) which was isolated from the patients with Mycosis fungoides by Gallo et al. [ Poiesz. B. J., et al. : Proc. Natl. Acad. Sci., 77, 7415 ( 1980 ) ] . It has also been known that HTLV - I in the free form may not be infectious and that it may turn to be infectious in cells through HTLV-I infected lymphocytes. Therefore, the limited pathway of it's infections such as infections through transfusion of blood containing peripheral lymphocytes from patients with adult T - cell leukemia or infections between husband and wife through sperm containing HTLV - I infected lymphocytes, have been confirmed.

However, the high incidence of the infections of HTLV -I from mothers to children have been reported and in these cases, it has been confirmed that the infections have been brought about through mother's milk containing lymphocytes infected with HTLV - I. There has been reported that the possibility of the infections in uterus or obstetric canal in the case of infections between mothers and children are substantially zero [Motohiko Ichijo et al. : Nihon Iji Shinpo, No. 3267 ( published in December 6, 1986 ), pages 11 - 14 ], while the infections through mother's milk have been evidenced. All lymphocyte samples prepared from the milk from postpartum mothers of 12, for example, have been detected of HTLV - I, which have been reported to have the infectious potential [Kinoshita, K., et al. : Gann, 75, 103 ( 1984), Nakano, S., et al. : ibid., 75, 1044 ( 1984 ) ].

For the protection from the HTLV - I infection through mother's milk, it has been recommended as one possibility to inert the infectious potential of HTLV - I in mother's milk with some suitable treatment, for example, treatment with heat, before using the milk for nursing babies ; however, the method to inactivate the infectious potential of HTLV I has not yet been established. On the other hand, it has been reported that the prohibition of breast - feeding followed by the conversion into an artificial feeding made the incidence of HTLV - I infections rapidly decrease [Motohiko Ichijo, et al. : Nihon Iji Shinpo, No. 3267 ( published in December 6, 1986 )]. The discontinuation of breast - feeding, which is not preferable because the nutritions in mother's milk may not be fed and the reationship between mothers and children may not be properly established, is the most reliable means to cut off the infectious pathway of HTLV - I to date.

It is required to provide an assay method that is useful to isolate subjects who should discontinue breast -feeding to change into artificial feeding from those who may still continue breast - feeding in order to establish the cut - off means as a routine method.

The method for detecting the presence of anti - HTLV -I antibodies in mother's serum is currently employed for the objective described above. That is, it is considered that a mother whose serum is shown positive for the antibody is a carrier of HTLV - I and her milk contains lymphocytes infected with HTLV - I having infectious potential. However, it should be further examined if the milk from the mother positive for anti - HTLV - I antibodies contains HTLV - I having infectious potential and another indicator should be employed to assess the possibility of containing HTLV - I wth infectious potential.

It is an object of the present invention to provide a means for detecting infections through mother's milk and reagents therefor.

[Means of solving the problems ]

As a result of making efforts to solve the problems, the present inventors have found that the antibodies to the antigen referred to as P40$^x$ are useful as an indicator for detecting the presence or the occurrence of the infection through mother's milk and achieved the present invention.

Namely, it is an object of the present invention to provide a method for detecting the presence or the occurrence of the HTLV - I infection from a mother to a child through the mother's milk, comprising an immunological reaction of the mother's serum to P40$^x$ using P40$^x$.

It is another object of the present invention to provide reagents for detecting the presence or the occurrence of HTLV - I infection from a mother to a child through the mother's milk comprising containing P40$^x$ as a vital element for constitution.

The present invention is explained in more detail as follows.

P40$^x$ is a part of the antigen protein of HTLV - I, and more particularly, it is a protein which has a molecular weight of about 40 Kd, among other proteins coded with the site of X region of HTLV - I gene.

P40$^x$ known to a person skilled in the art and has been described in the specification of EP-A-196 319 corresponding to USSN 652,775, wherein P40$^x$ under the name of P40$^{x1}$ is described of its properties, the process for its production and its usages. These descriptions are cited as reference in the present invention and P40$^x$ is provided from Triton Biosciences Inc. As for the chemical structure of P40$^x$, it is a protein having an amino acid sequence therein which is specified by $S_0$ described below aid furthermore, the amino acid sequence thereof has characteristic amino acid sequences in parts which are symbolized as $S_1$ - $S_4$.

$S_0$ PCLLSAHFPG FGQTLLFGYP VYVFGDCVQG

DWCPISGGLC SARLHRHALL ATCPEHQITW

DPIDGRVIGS ALQFLIPRLP SFPTQTTSKT

LKVLTPPITH TTPNIPPSFL QAMRKYSPFR

NGYMEPTLGQ HLPTLSFPDP GLRPQNLYTL

WGGSVVCMYL YQLSPPITWP LLPHVIFCHP

GQLGAFLTNV PYKRIEELLY KISLTTGALI

ILPEDCLPTT LFQPARAPVT LTAWQNGLLP

FHSTLTTPGL IWTFTDGTPM ISGPCPKDGQ

PSLVLQSSSF IFHKFQTKAY HPSFLLSHGL

IQYSSFHSLH LLFEEYTNIP ISLLFNEKEA

DDNDHEPQIS PGGLEPPSEK HFRETEV

$S_1$ CPEHQITW DPIDGR

$S_2$ KYSPFR NG

$S_3$ PDP GLRPQN

$S_4$ VLQSSSF IFHKFQTKAY HPSFLLSHGL

IQYSSFHSLH LLFEEYTNIP ISLLFNEKEA

DDNDHEPQIS PGGLEPPSEK HFRETEV

The codes of the amino acid sequence are:
P proline, C cysteine, L leucine, S serine, F phenylalanine, G glycine, Q glutamine, T threonine, Y tyrosine, V valine, D aspartic acid, N asparagine, W tryptophan, I isoleucine, A alanine, R arginine, H histidine, E glutamic acid, K lysine, M methionine.

The specification indicates that the amino acid sequence in the X region of HTLV - I is an antigen protein specific for HTLV - I and that a method for detecting HTLV - I infection may comprise detecting the antibodies to P40$^x$. However, it has not been made apparent whether or not the detection of the antibodies may be useful in detecting infection through mother's milk.

The immunological reaction in mother's serum for P40$^x$ is determined as an indicator of HTLV - I infection. Accordingly, as far as the antibodies in the serum has the property to show the antigen - antibody reaction,it should not be required to disclose the chemical and other physicochemical structure thereof. In addition, the immunological reaction may be determined by any other immunological detection method, all of which are included in the present invention.

In the present invention, the term " mother " means who provides breast milk to an unspecified child, that is, the child is not limited to her own.

The current evidence that the infection between a mother and a child may be caused through her milk have been assumed as a prepostion in the present invention. Thus, the present invention may propose a predictable information in order to protect children against the infection through mother's milk as well as an information about a potentiality such that the child infected with HTLV - I at present has had its pathway through mother's milk in the past. This means that the possible infection through mother's milk is determined based on the mother's serum positive for the immunological reaction to P40$^x$.

The method for detection in the present invention comprises the use of P40$^x$. Therefore, the reagents containing P40$^x$ as a vital element for constitution are defined as the measuring reagents themselves for use in an embodiment in the present invention. The measuring reagents have the same industrial field of utilization and problems to be solved as those of the detection method in the present invention.

Example

The present invention is illustrated in more detail in the following examples.

Example 1.

a) P40$^x$ ( manufactured by Triton Biosciences Inc. )
b) 0.05 M Tris - HCl buffer ( pH 8.0 ) containing 0.01 % sodium dodecyl sulfate ( SDS )
c) Physiological saline containing 0.01 % Tween 20
d) Monoclonal anti - human IgG ($\gamma$) antibody label led with alkaline phosphatase
e) P - nitrophenol phosphate 4 mg / ml
f) 1N NaOH

The measuring reagents in the present invention comprises the whole combination set of the above reagents described in a) - f).

Example 2.

Aliquots 100 $\mu$l of P40$^x$ , adjusted at a concentration of 0.25 $\mu$g / ml by 0.05 M Tris - HCl buffer pH 8.0 , containing 0.01 % SDS ) were poured into microtiter wells and left to stand at 4 °C overnight. Each well is washed with distilled water and 100 $\mu$l of each of normal rabbit serum is added to each well. These wells are divided in two ; to one half of the wells is added 20 $\mu$l of each of the control serum ( negative for anti - P40$^x$ antibody ), while 20 $\mu$l of each of the experimental serum is added to other half of the wells. Then, all wells are incubated at 37 °C for 60 min ( the 1st reaction ).

Each well is washed with physiological saline ( containing 0.01 % Tween 20 ) and 100 $\mu$l of each of monoclonal anti - human IgG ($\gamma$) antibodies label led with alkaline phosphatase is added thereto to incubate at 37 °C for 60 min ( the 2nd reaction ).

Each well is subsequently washed with physiological saline containing 0.01 % Tween 20 and then, the substrate solution 100 $\mu$l ( p - nitrophenol phosphate 4 mg / ml ) is added to each well to incubate at 37 °C for 30 min ( the 3rd reaction ).

To each well is added 100 $\mu$l of 1 N NaOH in order to terminate the reaction and the absorbance at 405 nm of the resulting solution is measured.

The absorbance five - fold that of the negative control ( the absorbance from which the absorbance of the reagents blank is subtracted ) is defined as the cut - off value ; the presence of immunological reaction to P40$^x$ is defined positive if the measured absorbance of a specimen shows the cut - off value or a value above it.

Effects of the invention

Experimental example 1.

The effects the present invention is explained in the following experimental examples.

a) Sample

Nine families whose mothers were found positive for anti - HTLV - I antibody by the detection method thereof described in the following section b), were studied and the respective sera of said mothers and childen thereof were used as samples.

b) Method

The presence of the immunological reaction to P40$^X$ in the serum of each mother is determined by the method described in example 2. Alternatively, the presence of anti - HTLV - I antibody in the children is determined by the assay method for anti - HTLV - I antibody described below.

[The assay method of anti - HTLV - I antibody ]

MT - 2 cells producing HTLV - I are solubilized in 0.15 M phosphate - buffered physiological saline ( PBS pH 7.2 containing 0.2 % sodium deoxycholate ). The resulting lysate is dialyzed against 0.05 M Tris - HCl buffer ( pH 8.0 ) and ultracentrifuged at 100,000 X g for 90 min. to obtain the resulting supernatant as a HTLV - I antigen solution. To each microtiter well is added 100 μl of the HTLV - I solution adjusted at 0.75 of OD$_{280}$ using 0.05 M Tris - HCl buffer pH 8.0 to leave at 4 °C overnight. Each well is washed with physiological saline. One hundred μl of each of normal rabbit serum is added to each well described herein. These wells are divided in two ; to one half of the wells is added 20 μl of each of the control serum negative for anti -HTLV - I, whereas an equal amount of each of the experimental serum is added to other half of the wells. All wells are incubated at 37 °C for 60 min ( the 1st reaction ).

Each well is washed with physiological saline and to each well described above is added 100 μl of each of monoclonal anti - human IgG (γ) antibodies labelled with alkaline phosphatase to incubate at 37 °C for 60 min ( the 2nd reaction ).

Each well is washed with physiological saline. The substrate solution 100 μl ( p - nitrophenyl phosphate 4 mg / ml ) is added to each well to incubate at 37 °C for 30 min ( the 3rd reaction ).

To each well is added 100 μl of 1 N NaOH in order to terminate the reaction and the absorbance at 405 nm of the resulting solution is measued.

The absorbance 2.5 - fold that of the negative control ( the absorbance after subtracting the blank absorbance ) is defined as the cut - off value ; the presence of HTLV - I antibodies is defined to be positive if the measured absorbance of a specimen shows the cut - off value or a value above it.

c) Result

Table 1 shows the results. In the table, the symbols ( + ) and (-) indicate the positiveness and the negativeness of the presence, respectively.

Table 1

| | | The presence of anti - HTLV - I antibody in child's serum | |
| | | + | - |
| The presence of immunological reaction to P40$^x$ in mother's serum | + ( n=4) | 3 ( 75.0 % ) | 1 ( 25.0 % ) |
| | - ( n=5) | 1 ( 20.0 % ) | 4 ( 80.0 % ) |

Experimental Example 2.

a) Sample and method

Twenty four families different from those described in experimental example 1, whose mothers were previously found positive for anti - HTLV - I antibody by the detection method thereof described in the following section b), were studied. Using the respective sera of said mothers and childen thereof as samples, the presence of HTLV - I antibody were determined by the method in Section b) described in experimental example 1.

Table 2

| | | The presence of anti – HTLV – I antibody in child's serum | |
| --- | --- | --- | --- |
| | | + | – |
| The presence of immunological reaction to P40$^x$ in mother's serum | + ( n=17) | 13 ( 76.5 % ) | 4 ( 23.5 % ) |
| | – ( n=7) | 0 ( 0.0 % ) | 7 (100.0 % ) |

Summary

Tables 1 and 2 indicate the following ;
Most of the children whose mothers were positive for anti - HTLV antibody and negative for the immunological reaction to P40$^x$, were found negative for anti - HTLV - I antibody, that is, they were not infected by HTLV - I ; on the other hand, most of the children whose mothers were positive for the immunological reaction to P40$^x$ were found positive for anti - HTLV - I antibody and this demonstrates they were infected by HTLV - I.

Generally, it has been accepted that most of the infections from mothers to children are occurred through the mother's milk and hence, it may be concluded that most of the children positive for HTLV - I described herein got infected through intake of the milk of the mothers thereof who were positive for the immunological reaction to P40$^x$.

Accordingly, the present results indicate that the assay of the presence of HTLV - I infection using the immunological reaction to P40$^x$ as an indicator may make it possible to know in advanve or follow up an infection through mother's milk.

Claims

1. A method for detecting the prescnce or the occurrence of HTLV - I infection from a mother to a child through the mother's milk, comprising detecting an immunological roaction of the mother's serum to P40$^x$ using P40$^x$.

2. Reagents for detecting the presence or the occurrence of HTLV - I infection from a mother to a child through the mother's milk, comprising containing P40$^x$ as a vital element for constitution.